# EUROPÄISCHE PATENTSCHRIFT

(11) **EP 0 888 273 B1**
(45) Veröffentlichungstag und Bekanntmachung des Hinweises auf die Patenterteilung: **05.09.2001**
(21) Anmeldenummer: 97906177.7
(22) Anmeldetag: 06.03.1997
(51) Int. Cl.: C07C 45/00, C07C 49/403, B01J 27/232, B01J 23/06

(54) **VERFAHREN ZUR DEHYDRIERUNG VON SEKUNDÄREN CYCLISCHEN ALKOHOLEN**
PROCESS FOR DEHYDROGENATING SECONDARY CYCLIC ALCOHOLS
PROCEDE DE DESHYDROGENATION D'ALCOOLS CYCLIQUES SECONDAIRES

(30) Priorität: 14.03.1996 DE 19609954
(43) Veröffentlichungstag der Anmeldung: 07.01.1999
(73) Patentinhaber: BASF AKTIENGESELLSCHAFT, 67056 Ludwigshafen (DE)
(72) Erfinder: BRÖCKER, Franz, Josef, D-67061 Ludwigshafen (DE); HESSE, Michael, D-67549 Worms (DE); MÄRKL, Robert, D-67136 Fussgönheim (DE)
(86) Internationale Anmeldenummer: EP9701124
(87) Internationale Veröffentlichungsnummer: WO9733853

(56) Entgegenhaltungen:
- GB-A- 1 054 617
- CHEMICAL ABSTRACTS, vol. 121, no. 22, 28.November 1994 Columbus, Ohio, US; abstract no. 258415, YANG L ET AL: "Studies on the structure and catalytic properties of ZnO/CaCO3 catalysts for cyclohexanol dehydrogenation" XP002032343 & SHIYOU HUAGONG (SHHUE8,10008144);93; VOL.22 (6); PP.370-4, NANJING UNIV.;DEP. CHEM.; NANJING; 210008; PEOP. REP. CHINA (CN),
- CHEMICAL ABSTRACTS, vol. 111, no. 14, 2.Oktober 1989 Columbus, Ohio, US; abstract no. 117268, YAO N ET AL: "Study on zinc-calcium catalyst for dehydrogenation of cyclohexanol by wide-angle x-ray diffraction" XP002032344 & SHIYOU HUAGONG (SHHUE8,10008144);89; VOL.18 (3); PP.191-6, HUBEI RES. INST. CHEM.;WUHAN; PEOP. REP. CHINA (CN),
- YANG ET AL: "Studies on the structure and catalytic properties of ZnO/CaCO3 catalysts for cyclohexanol dehydrogenation", PETROCHEMICAL ENGINEERING, , , Band 22, Nr. 6, Seiten 370 - 374
- YAO, N ET AL: "Study on Zinc-Calcium Catalyst for Dehydrogenation of cyclohexanol by wide-angle X-ray Diffraction", PETROCHEMICAL ENGINEERING, , , Band 18, Nr. 3, Seiten 191 - 196
- 'Gmelins Hanbuch Der Anorganischen Chemie', VERLAG CHEMIE GMBH, WEINHEIM/BERGSTRASSE

## Beschreibung

Die vorliegende Erfindung betrifft ein Verfahren zur Dehydrierung von sekundären Alkoholen in Gegenwart eines Katalysators, enthaltend Zinkoxid und Calciumcarbonat, bei erhöhter Temperatur in der Gasphase.

Aus der DE-A 1,443,462 ist ein Verfahren zur Dehydrierung von primären und sekundären Alkoholen bekannt, in dem der eingesetzte Alkohol bei erhöhter Temperatur in der Gasphase an vorwiegend aus Zinkoxid bestehenden Katalysatoren zum entsprechenden Aldehyd bzw. Keton dehydriert wird. Der Katalysator kann sowohl Kupferverbindungen als auch Erdalkalien enthalten. Während der Dehydrierung, d.h. nach dem Einsetzen der wasserstoffabspaltung, wird in dem beschriebenen Verfahren die Wasserstoffzufuhr eingestellt. Insbesondere wird die Dehydrierung von Cyclohexanol zu Cyclohexanon beschrieben, wobei die Ausbeute an Cyclohexanon allerdings nur 81,5% beträgt. Neben 17% unumgesetztem Cyclohexanol besteht das Reaktionsgemisch aus 0,1 bis 0,5% Kohlenwasserstoffen und 1% höhersiedenden Kondensationsprodukten.

Die DE-AS 1,296,625 beschreibt ein Verfahren zur Herstellung von Cyclohexanon aus mit organischen Säuren und Estern verunreinigtem Cyclohexanol bei erhöhten Temperaturen in Gegenwart eines zinkhaltigen Katalysators, bestehend aus Zinkoxid-Zinkcarbonat oder Mischungen von Zinkoxid-Zinkcarbonat mit Calciumoxid-Calciumcarbonat oder mit Magnesiumoxid-Magnesiumcarbonat. Nachteilig an diesem Verfahren ist die zu starke Abnahme der Tablettenhärte im Dauerbetrieb, was zu häufigem Auswechseln des Katalysators und entsprechenden Ausfallzeiten führt. Die Abnahme der Tablettenhärte im Dauerbetrieb erfolgt durch die massive Zersetzung der Carbonate durch organische Säuren bzw. durch Phasenumwandlungen.

Aus Acta Chim. Acad. Sci. Hung 107 (1981) 343-360 als auch aus Acta Chim. Acad. Sci. Hung 97 (1978) 439-449 ist bekannt, daß bei der Dehydrierung von Cyclohexanol in Gegenwart von Wasserstoff und Katalysatoren, die Elemente der achten Nebengruppe wie Rhodium, Nickel und Platin enthalten, im Gegensatz zur Verfahrensweise ohne Wasserstoff vermehrt Crackprodukte und Bildung von Phenol und Benzol zu beobachten ist. Dies wird bei der Dehydrierung aliphatischer Alkohole nicht beobachtet: so beschreibt die DE-A 2,028,350 ein Verfahren zur Dehydrierung von Aldehyden und Ketonen, insbesondere zur Herstellung von Aceton und Methylisobutylketon, an einem Kupfer-haltigen Katalysator in Gegenwart von Wasserstoff. Nebenbei sei angemerkt, daß zwar Cyclohexanol als Ausgangsprodukt unter vielen anderen mitaufgeführt wird, jedoch liegen keine experimentellen Daten zur Dehydrierung von Cyclohexanol vor. Man wird nach dem in der DE-A 2,028,350 beschriebenen Verfahren aller Voraussicht nach auch Cyclohexanon herstellen können, jedoch wird man im Lichte der o.g. Acta Chim. Acad. Sci. Hung 107 (1981) 343-360 als auch Acta Chim. Acad. Sci. Hung 97 (1978) 439-449 mit Nebenprodukten zu rechnen haben, die eine wirtschaftliche Anwendung verbieten.

Die Aufgabe der vorliegenden Erfindung bestand daher darin, ein Verfahren zur Verfügung zu stellen, bei dem cyclische Ketone, insbesondere Cyclohexanon, in höheren Selektivitäten und Ausbeuten als bislang möglich erhalten werden können, und bei dem die Bildung von Crackprodukten und aromatischen Nebenprodukten minimiert wird. Des weiteren sollte ein Katalysator zur Verfügung gestellt werden, der im Dauerbetrieb eine gute Tablettenhärte, insbesondere mit Bezug auf Stirndruckfestigkeit und Seitendruckfestigkeit, aufweist, so daß ein Katalysatorwechsel weniger oft als bislang durchgeführt werden muß.

Demgemäß wurde ein Verfahren zur Dehydrierung von sekundären Alkoholen in Gegenwart eines Katalysators, enthaltend Zinkoxid und Calciumcarbonat, bei erhöhter Temperatur in der Gasphase gefunden, indem man sekundäre cyclische Alkohole einsetzt, die Dehydrierung in Gegenwart von Wasserstoff durchführt, und einen Katalysator einsetzt, dessen aktive Komponenten von 30 bis 60 Gew.-% aus Zinkoxid und von 40 bis 70 Gew.-% aus Calciumcarbonat in der Calcit-Modifikation besteht.

Des weiteren wurde ein Dehydrierungskatalysator sowie ein Verfahren zu seiner Herstellung und seine Verwendung gefunden.

Als sekundäre Alkohole kann man erfindungsgemäß cycloaliphatische Alkohole mit 5 bis 16 Kohlenstoffatomen wie Cyclopentanol, Cyclohexanol, 4-Methylcyclohexanol, Cyclooctanol, Cyclododecanol und Cyclohexadecanol, bevorzugt Cyclohexanol, einsetzen.

Als Zinkoxid-haltigen Katalysator setzt man erfindungsgemäß einen Katalysator ein, dessen aktive Komponenten von 30 bis 60, bevorzugt von 40 bis 50 Gew.-% aus Zinkoxid und von 40 bis 70, bevorzugt von 50 bis 60 Gew.-% aus Calciumcarbonat in der Calcit-Modifikation besteht.

In einer bevorzugten Ausführungsform weist der erfindungsgemäße Katalysator eine spezifische Oberfläche nach BET von 5 bis 50, vorzugsweise von 10 bis 30 m²/g auf.

Ein solcher Katalysator ist erfindungsgemäß durch Ausfällen von schwerlöslichen Zink- und Calciumverbindungen aus wasserlöslichen Zink- und Calciumverbindungen mit einer Base und anschließende Aufarbeitung in an sich bekannter Weise erhältlich, wobei man
(a) als Base ein wasserlösliches basisches Carbonat einsetzt,
(b) gewünschtenfalls die schwerlöslichen Zink- und Calciumverbindungen nach dem Ausfällen filtriert,
(c) die gewünschtenfalls filtrierten Zink- und Calciumverbindungen wäscht,
(d) die gewaschenen Zink- und Calciumverbindungen aus (c) trocknet unter Erhalt eines Pulvers, und anschließend
(e) das Pulver aus (d) bei Temperaturen von nicht über 600°C calciniert, und
(f) gewünschtenfalls das calcinierte Pulver zu Formkörpern verpreßt.

Als wasserlösliche Zink- und Calciumsalze kann man Acetate, Sulfate, Nitrate, bevorzugt Nitrate wie Zinknitrat, Zinkacetat, Zinksulfat, Calciumacetat, Calciumnitrat, bevorzugt Zinknitrat und Calciumnitrat, einsetzen. Üblicherweise kann man wäßrige Lösungen der entsprechenden Salze in Konzentrationen im Bereich von 3 bis 25, bevorzugt von 10 bis 25, insbesondere 20 Gew.-%, einsetzen.

Das Molverhältnis von Zink zu Calcium wählt man so, daß nach dem Calcinieren die aktiven Komponenten des Katalysators von 30 bis 60 Gew.-% aus Zinkoxid und 40 bis 70 Gew.-% aus Calciumcarbonat in der Calcit-Modifikation vorliegen.

Als Base verwendet man wasserlösliche basische Carbonate wie Alkalimetallcarbonate wie Natriumcarbonat, Kaliumcarbonat, Alkalimetallhydrogencarbonate wie Natriumhydrogencarbonat, Kaliumhydrogencarbonat, Ammoniumcarbonat oder Ammoniumhydrogencarbonat sowie deren Mischungen, vorzugsweise Natriumcarbonat, besonders bevorzugt in Form ihrer wäßrigen Lösungen in Konzentrationen im allgemeinen im Bereich von 0,5 bis 30, bevorzugt von 10 bis 25 Gramm Base/100 Gramm Lösung.

Die Fällung führt man im allgemeinen bei Temperaturen im Bereich von 10 bis 90, vorzugsweise von 40 bis 80°C durch. Nach dem Ausfällen kann man gewünschtenfalls den Niederschlag abfiltrieren.

Der gewünschtenfalls abfiltrierte Niederschlag wäscht man in der Regel mit Wasser, bevorzugt solange, bis kein Nitrat mehr mittels der Nitratringprobe feststellbar ist, und trocknet ihn anschließend bevorzugt bei einer Temperatur im Bereich von 90 bis 150°C unter Erhalt eines getrockneten Pulvers. Die Trocknung kann in ruhender oder bewegter Schicht, vorzugsweise durch Sprühtrocknung, erfolgen.

Das getrocknete Pulver wird erfindungsgemäß bei Temperaturen von nicht höher als 600°C, bevorzugt im Bereich von 300 bis 600°C, insbesondere von 400 bis 475°C, bevorzugt in Luft, calciniert. Nach bisherigen Beobachtungen führt eine längere Erhitzung über 600°C zur Bildung der Aragonit-Modifikation von CaCO₃. Eine kurzfristige Erhitzung über 600°C ist dann nicht hinderlich für die Herstellung der erfindungsgemäßen Katalysatoren, solange sich dabei kein Aragonit (d.h. durch Nachweis mittels Röntgendiffraktometrie) bildet.

Nach dem Calcinieren kann man gewünschtenfalls das calcinierte Pulver zu Formkörpern wie Tabletten, Ringe, Zylinder etc., bevorzugt Tabletten, verpressen.

In einer bevorzugten Ausführungsform verpreßt man das calcinierte Pulver zusammen mit Graphit, bevorzugt mit 0,1 bis 5, besonders bevorzugt mit 1 bis 2,5, insbesondere 2 Gew.-%, bezogen auf die Gesamtmasse, Graphit.

In einer weiteren bevorzugten Ausführungsform verpreßt man das uncalcinierte Pulver aus Schritt (c) (s.o.) zu Formkörpern, bevorzugt zu Tabletten, und calciniert die so erhaltenen Formkörper wie unter Schritt (d) beschrieben.

Die so erhaltenen calcinierten Pulver und Formkörper können als Katalysatoren eingesetzt werden, wobei diese Katalysatoren als aktive Komponenten Zinkoxid und Calciumcarbonat (in der Calcit-Modifikation) und als passive Komponente gewünschtenfalls Graphit enthalten.

Die erfindungsgemäßen Katalysatoren weisen folgende physikalische Eigenschaften auf:

In einer weiteren bevorzugten Ausführungsform setzt man einen Katalysator der erfindungsgemäßen Art ein, der ein Porenvolumen im Bereich von 0,10 bis 0,50, insbesondere von 0,20 bis 0,35 cm³/g, bei einem Porendurchmesser im Bereich von 5 nm bis 300 µm aufweist, wobei besonders bevorzugt mindestens 85 %, vorzugsweise mehr als 90 % dieses Porenvolumens mit einem Porendurchmesser im Bereich von 0,01 bis 0,5 µm verbunden ist.

Besonders bevorzugte Katalysatoren der genannten Art sind solche, die eine Stirndruckfestigkeit im Bereich von 500 bis 4000 N/cm², insbesondere von 1000 bis 2500 N/cm² und eine Seitendruckfestigkeit von 30 bis 300 N, vorzugsweise 50 bis 200 N aufweisen. Diese Werte lassen sich auch ohne Calcination erzielen. Wichtig ist, daß unter Betriebsbedingungen (Reaktionsbedingungen) diese angegebenen Festigkeitsbereiche erhalten bleiben. Dies ist jedoch nur der Fall, wenn keine Phasenumwandlungen nachfolgen. Durch das erfindungsgemäße Verfahren ist diese Bedingung gegeben.

Die spezifische Oberfläche nach BET beträgt im allgemeinen 5 bis 50 m²/g, vorzugsweise 10 bis 30 m²/g. Das Porenvolumen im Porendurchmesserbereich zwischen 5 nm und 300 µm besitzt Werte üblicherweise zwischen 0,1 und 0,5 cm³/g, vorzugsweise 0,2 bis 0,35 cm³/g mit der Maßgabe, daß mindestens 85 %, vorzugsweise mehr als 90 % dieses Porenvolumens im Porendurchmesserbereich von 0,01 bis 0,5 µm liegen.

Die Stirndruckfestigkeit der Tabletten beträgt vorzugsweise 500 bis 4000 N/cm², insbesondere 1000 bis 2500 N/cm² und die Seitendruckfestigkeit der Pillen liegt bevorzugt zwischen 30 und 300 N, vorzugsweise 50 bis 200 N.

In einer besonders bevorzugten Ausführungsform wäscht man den Niederschlag aus schwerlöslichen Zink- und Calciumverbindungen, bevorzugt Zinkhydroxidcarbonat und Calciumcarbonat, auf Filterpressen, maischt den dabei erhaltenen Filterkuchen mit Wasser an, und versprüht die Maische zum Trocknen in einem Sprühturm. Das auf diese Weise erhaltene getrocknete Sprühpulver kann man danach wie oben beschrieben weiterverarbeiten.

Erfindungsgemäß bringt man den gasförmigen sekundären, cyclischen Alkohol, bevorzugt Cyclohexanol, dem 1 bis 20, vorzugsweise 5 bis 10 Vol.-% Wasserstoff, bezogen auf die Menge an Alkohol, zugemischt sind, in Kontakt mit dem eingesetzten Katalysator in an sich üblicher Weise, beispielsweise in einem Festbettreaktor oder in einem Wirbelschichtreaktor, bevorzugt in einem Rohrreaktor, in dem der Katalysator fest angeordnet ist. Der Austrag wird üblicherweise destillativ aufgearbeitet.

Im allgemeinen verdampft man den einzusetzenden Alkohol in an sich bekannter Weise, beispielsweise in einem Verdampfer, und mischt dann die gewünschte Menge an gasförmigem Wasserstoff zu.

Die Temperatur der Gasphase in der Reaktionszone wählt man üblicherweise im Bereich von 200 bis 500, vorzugsweise von 300 bis 450°C. In einer bevorzugten Ausführungsform wählt man den Temperaturbereich so, daß man einen Umsatz im Bereich von 50 bis 90, bevorzugt von 65 bis 75 %, Alkohol erhält. Im Falle von Cyclohexanol als Ausgangsverbindung wählt man dann die Temperatur im Bereich von 350 bis 400°C.

Den Druck der Gasphase in der Reaktionszone wählt man im allgemeinen im Bereich von 80 bis 4000, vorzugsweise von 100 bis 1000 kPa.

Die Belastung des Katalysators wählt man im allgemeinen im Bereich von 0,5 bis 3,0, vorzugsweise von 0,6 bis 2,0 Liter Alkohol pro Liter an Katalysator und pro Stunde.

In einer bevorzugten Ausführungsform trennt man aus dem die Reaktionszone verlassenden Reaktionsgemisch den Wasserstoff ab und setzt ihn der die Reaktionszone eintretenden Gasmischung zu.

Die erfindungsgemäß hergestellten Ketone wie Cyclohexanon sind wichtige großindustrielle Produkte. Beispielsweise wird Cyclohexanon üblicherweise, bevorzugt im anfallenden Gemisch mit Cyclohexanol, zur Herstellung von Adipinsäure weiterverwendet.

Der Vorteil des erfindungsgemäßen Verfahrens liegt darin, daß cyclische Ketone, insbesondere Cyclohexanon, in höheren Ausbeuten als bislang möglich erhalten werden kann, und bei dem die Bildung von Crackprodukten und aromatischen Nebenprodukten minimiert wird.

### Beispiele

### Beispiel 1 - Herstellung eines calcinierten Katalysators (K1ₒ)

Für die Herstellung des Katalysators werden zwei Lösungen benötigt. Lösung 1 ist eine wäßrige Lösung von Zinknitrat und Calciumnitrat mit einer Konzentration von 20 Gew.-%, in der das Molverhältnis von Zink:Calcium = 1 : 1,6 ist. Lösung 2 ist eine zweimolare wäßrige Natriumcarbonatlösung.

Beide Lösungen werden auf 70°C erhitzt und parallel in einen Fällungsbehälter gepumpt. Dabei wird der Zulauf der Lösungen so geregelt, daß während der Fällung ein pH-Wert von 7,8 ± 1,0 aufrechterhalten wird. Der bei dieser Parallelfällung entstandene Niederschlag wird abfiltriert und mit Wasser solange gewaschen bis kein Nitrat mehr nachweisbar ist (Test mit FeSO₄-Lösung und konz. H₂SO₄, sogenannte Nitratringprobe). Anschließend wird der Niederschlag in Wasser angemaischt und sprühgetrocknet. Das so erhaltene Pulver wird 5 h bei 450°C an der Luft erhitzt und nach Abkühlung und Zusatz von 2 Gew.-% Graphit zu 5x5 mm Tabletten verpreßt. Die physikalischen Daten zur Charakterisierung des Katalysators ("K1ₒ") sind in Tab. 1 aufgelistet.

### Beispiel 2 - Dehydrierung mit K1ₒ

920 g des nach Beispiel 1 hergestellten Katalysators K1ₒ wurden in einen Rohrreaktor mit einer Länge von 0,6 m und einem Innendurchmesser von 0,05 m eingebaut. Über einen Verdampfer wurden 640 ml/h flüssiges Cyclohexanol dem Reaktor gasförmig zugeführt. Vor dem Reaktor wurden 7 l/h Wasserstoff zudosiert. Die Temperatur des Reaktionsgemisches in der Reaktionszone wurde auf 331°C gehalten. Bei dieser Temperatur betrug der Umsatz, bezogen auf eingesetztes Cyclohexanol, 70 %. Das den Reaktor verlassende Reaktionsgemisch wurde auf Raumtemperatur abgekühlt unter Freisetzung von Wasserstoff. Die flüssigen Reaktionsprodukte wurden gaschromatographisch analysiert. Bei einem Umsatz von 70 % wurden nach 1800 h eine Selektivität von 99,0 % und einen Rückstand von 0,70 % erhalten. Der Katalysator (K1₁₈₀₀) hatte die in Tab. 1 angegebenen physikalischen Eigenschaften.

### Vergleichsbeispiel 1 - Herstellung eines nicht calcinierten Katalysators und Dehydrierung damit

Es wurde, wie in Beispiel 1 beschrieben, aus einer wäßrigen Zink- und Calciumnitratlösung mittels 2 molarer Natriumcarbonatlösung Calciumcarbonat und Zinkhydroxidcarbonat ausgefällt. Der Niederschlag wurde nitratfrei gewaschen und nach Anmaischen mit H₂O sprühgetrocknet. Das so erhaltene trockene Pulver wurde nach Zusatz von 2 Gew.-% Graphit zu 5x5 mm Pillen verpreßt. Der so erhaltene Katalysator ("K2ₒ") besitzt die in Tabelle 1 angegebenen physikalischen Daten. Die Röntgenanalyse zeigt als Hauptmenge Calcit und als Nebenmengen Aragonit und Zinkhydroxidcarbonat. 920 g dieses Katalysators wurden, wie in Beispiel 2 beschrieben, bei einem Cyclohexanolumsatz von 70 % getestet. Die Selektivität des Katalysators betrug nach 1800 h 98,5 % und die gebildete Rückstandsmenge 0,88 %. Der Katalysator (K2₁₈₀₀) hatte die in Tabelle 1 angegebenen physikalischen Eigenschaften.

### Beispiel 3 - Dehydrierung mit K1ₒ (140 h)

920 g des nach Beispiel 1 hergestellten Katalysators (K1₀) wurden in einen Rohrreaktor mit einer Länge von 0,6 m und einem Innendurchmesser von 0,05 m eingebaut. Über einen Verdampfer wurden 640 ml/h flüssiges Cyclohexanol dem Reaktor gasförmig zugeführt. Vor dem Reaktor wurden 7 l/h Wasserstoff zudosiert. Die Temperatur in der Reaktionszone wurde auf 341°C gehalten. Bei dieser Temperatur betrug der Umsatz, bezogen auf eingesetztes Cyclohexanol, 70 %. Das den Reaktor verlassende Reaktionsgemisch wurde auf Raumtemperatur abgekühlt unter Freisetzung von Wasserstoff. Die flüssigen Reaktionsprodukte wurden gaschromatographisch analysiert. Bei einem Umsatz von 70 % wurden nach 140 h eine Selektivität von 98,7 % und einen Rückstand von 0,80 % erhalten. Der Katalysator wurde ausgebaut (K1₁₄₀) und hatte die in Tabelle 1 angegebenen physikalischen Eigenschaften.

### Vergleichsbeispiel 2 - Dehydrierung mit K2ₒ, ohne Wasserstoff

Beispiel 3 wurde wiederholt mit Katalysator (K2₀) aus Vergleichsbeispiel 1 mit den weiteren Unterschieden, daß kein Wasserstoff eingesetzt wurde und die Reaktionstemperatur 323°C und die Versuchsdauer 143 h betrug. Die Selektivität betrug bei einem Umsatz von 70 %, bezogen auf eingesetztes Cyclohexanol, 96,6 %, der Rückstand betrug 2,37 %. Der ausgebaute Katalysator (K2₁₄₃) hatte die in Tabelle 1 angegebenen physikalischen Eigenschaften.

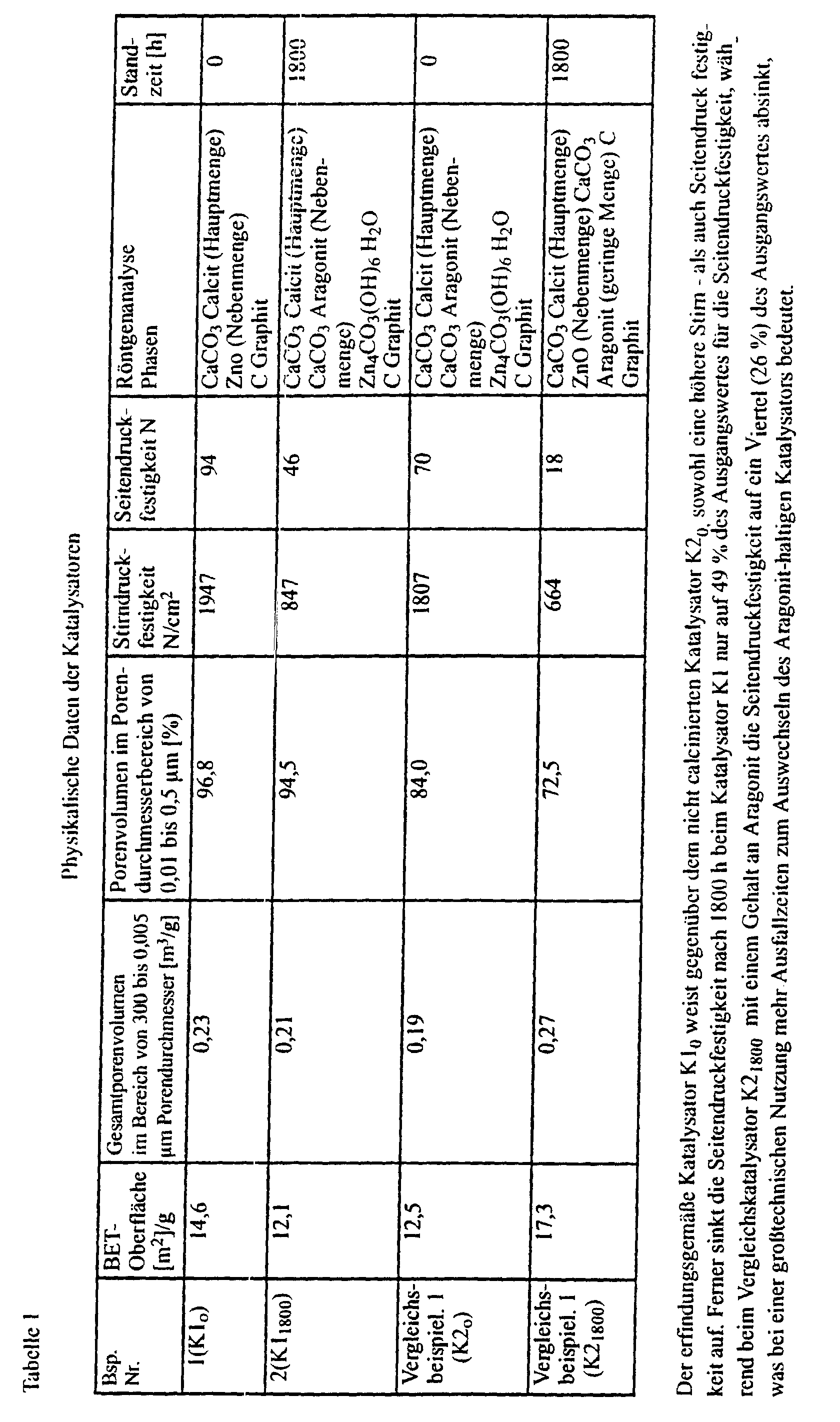

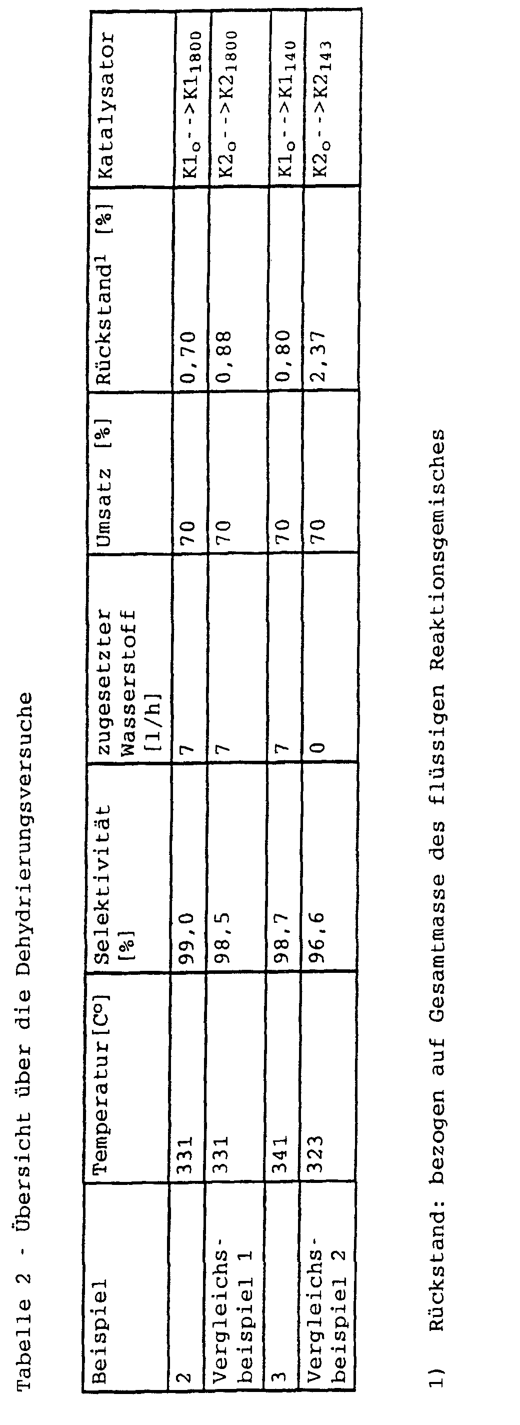

## Patentansprüche

1. Verfahren zur Dehydrierung von sekundären Alkoholen in Gegenwart eines Katalysators, enthaltend Zinkoxid und Calciumcarbonat, bei erhöhter Temperatur in der Gasphase, **dadurch gekennzeichnet, daß** man sekundäre cyclische Alkohole einsetzt, die Dehydrierung in Gegenwart von Wasserstoff durchführt, und einen Katalysator einsetzt, dessen aktive Komponenten von 30 bis 60 Gew.-% aus Zinkoxid und von 40 bis 70 Gew.-% aus Calciumcarbonat in der Calcit-Modifikation besteht.

2. Verfahren nach Anspruch 1, **dadurch gekennzeichnet**, daß man als sekundären cyclischen Alkohol Cyclohexanol einsetzt.

3. Verfahren gemäß den Ansprüchen 1 oder 2, **dadurch gekennzeichnet**, daß der eingesetzte Katalysator eine spezifische Oberfläche nach BET im Bereich von 5 bis 50 m²/g aufweist.

4. Verfahren gemäß den Ansprüchen 1 bis 3, **dadurch gekennzeichnet**, daß der eingesetzte Katalysator ein Porenvolumen bei einem Porendurchmesser im Bereich von 5 nm bis 300 µm von 0,1 bis 0,5 cm³/g aufweist.

5. Verfahren gemäß Anspruch 4, **dadurch gekennzeichnet**, daß mindestens 85 % des Porenvolumens Porendurchmesser im Bereich von 0,01 bis 0,5 µm aufweisen.

6. Dehydrierungskatalysator mit den aktiven Komponenten aus 30 bis 60 Gew.-% Zinkoxid und von 40 bis 70 Gew.-% Calciumcarbonat in der Calcit-Modifikation.

7. Dehydrierungskatalysator gemäß Anspruch 6, **dadurch gekennzeichnet**, daß der eingesetzte Katalysator eine spezifische Oberfläche nach BET im Bereich von 5 bis 50 m²/g aufweist.

8. Dehydrierungskatalysator nach den Ansprüchen 6 oder 7, **dadurch gekennzeichnet, daß** der Katalysator ein Porenvolumen bei einem Porendurchmesser im Bereich von 5 nm bis 300 µm von 0,10 bis 0,50 cm³/g aufweist.

9. Dehydrierungskatalysator nach den Ansprüchen 6, 7 oder 8, **dadurch gekennzeichnet, daß** mindestens 85% des Porenvolumens des Katalysators einen Porendurchmesser im Bereich von 0,01 bis 0,5 µm aufweisen.

10. Verfahren zur Herstellung des Dehydrierungskatalysator gemäß Anspruch 6 durch Ausfällen von schwerlöslichen Zink- und Calciumverbindungen aus wasserlöslichen Zink- und Calciumsalzlösungen mit einer Base und anschließende Aufarbeitung in an sich bekannter Weise, **dadurch gekennzeichnet**, daß man
(a) als Base ein wasserlösliches basisches Carbonat einsetzt,
(b) gewünschtenfalls die schwerlöslichen Zink- und Calciumverbindungen nach dem Ausfällen filtriert,
(c) die gewünschtenfalls filtrierten Zink- und Calciumverbindungen wäscht,
(d) die gewaschenen Zink- und Calciumverbindungen aus (c) trocknet unter Erhalt eines Pulvers, und anschließend
(e) das Pulver aus (d) bei Temperaturen von nicht über 600°C calciniert, und
(f) gewünschtenfalls das calcinierte Pulver zu Formkörpern verpreßt.

11. Verfahren zur Herstellung des Dehydrierungskatalysators nach Anspruch 10, **dadurch gekennzeichnet**, daß man nach Schritt (c) und vor Schritt (d) das Pulver zu Formkörpern verpreßt und anschließend bei Temperaturen von nicht über 600°C calciniert.

12. Verfahren nach Anspruch 10, **dadurch gekennzeichnet**, daß man den Niederschlag an schwerlöslichen Zink- und Calciumverbindungen auf Filterpressen wäscht, anschließend den dabei erhaltenen Filterkuchen mit Wasser anmaischt, dann die Maische zum Trocknen in einem Sprühturm versprüht, und danach das erhaltene Pulver wie unter (d) und gewünschtenfalls (e) beschrieben weiterbearbeitet.

13. Verwendung des Dehydrierungskatalysators gemäß den Ansprüchen 6 bis 9 oder hergestellt gemäß den Ansprüchen 10 bis 12 zur Dehydrierung von sekundären cyclischen Alkoholen.

14. Dehydrierungskatalysator nach den Ansprüchen 6 bis 9 oder hergestellt nach den Ansprüchen 10 bis 12, **dadurch gekennzeichnet**, daß er in Tablettenform eine Stirndruckfestigkeit im Bereich von 500 bis 4000 N/cm² und eine Seitendruckfestigkeit im Bereich von 30 bis 300 N aufweist.

15. Dehydrierungskatalysator nach Anspruch 14, **dadurch gekennzeichnet**, daß man durch Röntgendiffraktometrie des Dehydrierungskatalysators nur Calcit und Zinkoxid nachweisen kann.

## Claims

1. A process for dehydrogenating secondary alcohols in the presence of a catalyst comprising zinc oxide and calcium carbonate at elevated temperature in the gas phase, wherein secondary cyclic alcohols are used and the dehydrogenation is carried out in the presence of hydrogen and a catalyst whose active components comprise from 30 to 60 % by weight of zinc oxide and from 40 to 70 % by weight of calcium carbonate in the calcite modification.

2. A process as claimed in claim 1, wherein the secondary cyclic alcohol used is cyclohexanol.

3. A process as claimed in claim 1 or 2, wherein the catalyst used has a BET specific surface area in the range from 5 to 50 m²/g.

4. A process as claimed in any of claims 1 to 3, wherein the catalyst used has a pore volume at a pore diameter in the range from 5 nm to 300 µm of from 0.1 to 0.5 cm³/g.

5. A process as claimed in claim 4, wherein at least 85 % of the pore volume has a pore diameter in the range from 0.01 to 0.5 µm.

6. A dehydrogenation catalyst having the active components comprising from 30 to 60 % by weight of zinc oxide and from 40 to 70 % by weight of calcium carbonate in the calcite modification.

7. A dehydrogenation catalyst as claimed in claim 6 having a BET specific surface area in the range from 5 to 50 m²/g.

8. A dehydrogenation catalyst as claimed in claim 6 or 7 having a pore volume at a pore diameter in the range from 5 nm to 300 µm of from 0.10 to 0.50 cm³/g.

9. A dehydrogenation catalyst as claimed in claims 6, 7 or 8, wherein at least 85 % of the pore volume of the catalyst has a pore diameter in the range from 0.01 to 0.5 µm.

10. A process for preparing the dehydrogenation catalyst as claimed in claim 6 by precipitation of sparingly soluble zinc and calcium compounds from water-soluble zinc and calcium salt solutions using a base and subsequent work-up in a manner known per se, wherein
(a) the base used is a water-soluble basic carbonate,
(b) if desired, the sparingly soluble zinc and calcium compounds are filtered off after precipitation,
(c) the zinc and calcium compounds, which may have been filtered off, are washed,
(d) the washed zinc and calcium compounds from (c) are dried to give a powder, and subsequently
(e) the powder from (d) is calcined at not above 600°C, and
(f) if desired, the calcined powder is pressed to give shaped bodies.

11. A process for preparing the dehydrogenation catalyst as claimed in claim 10, wherein after step (c) and before step (d) the powder is pressed to give shaped bodies and is subsequently calcined at not above 600°C.

12. A process as claimed in claim 10, wherein the precipitate of sparingly soluble zinc and calcium compounds is washed on filter presses, the resulting filter cake is subsequently slurried with water, the slurry is then dried by spraying in a spray drier and the powder obtained is then further processed as described under (e) and, if desired, (f).

13. Use of the dehydrogenation catalyst as claimed in any of claims 6 to 9 or prepared as claimed in any of claims 10 to 12 for the dehydrogenation of secondary cyclic alcohols.

14. A dehydrogenation catalyst as claimed in any of claims 6 to 9 or prepared as claimed in any of claims 10 to 12, which in pellet form has a compressive strength on the end face in the range from 500 to 4000 N/cm² and a lateral compressive strength in the range from 30 to 300 N.

15. A dehydrogenation catalyst as claimed in claim 14, wherein only calcite and zinc oxide can be detected by X-ray diffractometry on the dehydrogenation catalyst.

## Revendications

1. Procédé de déshydrogénation d'alcools secondaires en présence d'un catalyseur, contenant de l'oxyde de zinc et du carbonate de calcium, à température élevée et en phase gazeuse, **caractérisé en ce que** l'on met en oeuvre des alcools secondaires cycliques, que l'on entreprend la déshydrogénation en présence d'hydrogène et que l'on met en oeuvre un catalyseur dont le composant actif est constitué de 30 à 60% en poids d'oxyde de zinc et de 40 à 70% en poids de carbonate de calcium sous la forme de calcite.

2. Procédé selon la revendication 1, **caractérisé en ce que** l'on met en oeuvre du cyclohexanol en tant qu'alcool secondaire cyclique.

3. Procédé selon l'une des revendications 1 ou 2, **caractérisé en ce que** le catalyseur mis en oeuvre présente une surface spécifique BET de 5 à 50 m²/g.

4. Procédé selon les revendications 1 à 3, **caractérisé en ce que** le catalyseur mis en oeuvre présente un volume porique de 0,1 à 0,5 cm³/g pour un diamètre porique de l'ordre de 5 nm à 300 µm.

5. Procédé selon la revendication 4, **caractérisé en ce qu**'au moins 85% du volume porique présentent des diamètres poriques de l'ordre de 0,01 à 0,5 µm.

6. Catalyseur de déshydrogénation comportant un composant actif constitué de 30 à 60% en poids d'oxyde de zinc et de 40 à 70% en poids de carbonate de calcium sous la forme de calcite.

7. Catalyseur de déshydrogénation selon la revendication 6, **caractérisé en ce que** le catalyseur mis en oeuvre présente une surface spécifique BET de l'ordre de 5 à 50 m²/g.

8. Catalyseur de déshydrogénation selon l'une des revendications 6 ou 7, **caractérisé en ce que** le catalyseur présente un volume porique de 0,10 à 0,50 cm³/g pour un diamètre porique de l'ordre de 5 nm à 300 µm.

9. Catalyseur de déshydrogénation selon les revendications 6, 7 ou 8, **caractérisé en ce qu**'au moins 85% du volume porique du catalyseur présentent un diamètre porique de l'ordre de 0,01 à 0,5 µm.

10. Procédé de préparation du catalyseur de déshydrogénation selon la revendication 6 par précipitation de composés peu solubles dans l'eau de zinc et de calcium à partir de solutions de zinc et de calcium solubles dans l'eau, au moyen d'une base, et retraitement subséquent par un procédé en soi connu, **caractérisé en ce que**
(a) on met en oeuvre un carbonate basique soluble dans l'eau en tant que base,
(b) on filtre éventuellement les composés peu solubles de zinc et de calcium après précipitation,
(c) on lave les composés de zinc et de calcium éventuellement filtrés,
(d) on sèche les composés de zinc et de calcium lavés de l'étape (c) de manière à obtenir une poudre, et ensuite
(e) on calcine la poudre obtenue en (d) à des températures non supérieures à 600°C, et
(f) On presse éventuellement la poudre calcinée pour obtenir des corps façonnés.

11. Procédé de préparation du catalyseur de déshydrogénation selon la revendication 10, **caractérisé en ce qu**'après l'étape (c) et avant l'étape (d), on presse la poudre pour obtenir des corps façonnés que l'on calcine ensuite à des températures non supérieures à 600°C.

12. Procédé selon la revendication 10, **caractérisé en ce que** l'on lave le précipité de composés de zinc et de calcium peu solubles dans l'eau sur des filtres-presses, que le gâteau de filtration ainsi obtenu est ensuite mouillé à l'eau, après quoi le mélange est pulvérisé aux fins de séchage dans une tour de pulvérisation, et la poudre obtenue est ensuite retraitée comme décrit en (d) et éventuellement en (e).

13. Utilisation du catalyseur de déshydrogénation selon les revendications 6 à 9 ou préparé selon les revendications 10 à 12 pour la déshydrogénation d'alcools secondaires cycliques.

14. Catalyseur de déshydrogénation selon les revendications 6 à 9 ou préparé selon les revendications 10 à 12, **caractérisé en ce qu**'il présente, sous sa forme en pastilles, une résistance à la compression frontale de l'ordre de 500 à 4000 N/cm² et une résistance à la compression latérale de l'ordre de 30 à 300 N/cm².

15. Catalyseur de déshydrogénation selon la revendication 14, **caractérisé en ce que** la diffractométrie aux rayons X du catalyseur de déshydrogénation ne met en évidence que de la calcite et de l'oxyde de zinc.
